# EUROPEAN PATENT SPECIFICATION

(11) **EP 3 393 535 B1**
(45) Date of publication and mention of the grant of the patent: **28.07.2021**
(21) Application number: 16823427.6
(22) Date of filing: 19.12.2016
(51) Int. Cl.: A61L 27/36

(54) **METHODS FOR PREPARING DRY CROSS-LINKED TISSUE**
VERFAHREN ZUR HERSTELLUNG VON TROCKENEM VERNETZTEM GEWEBE
PROCÉDÉS DE PRÉPARATION D'UN TISSU BIOLOGIQUE RÉTICULÉ SEC

(30) Priority: 21.12.2015 US 201514976092
(43) Date of publication of application: 31.10.2018
(73) Proprietor: Medtronic Vascular Inc., Santa Rosa, CA 95403 (US)
(72) Inventor: WANG, Wei, Santa Rosa, CA 95403 (US); GARDE, Kshitija, Santa Rosa, CA 95403 (US); GUO, Ya, Santa Rosa, CA 95403 (US); WONG, Benjamin, Santa Rosa, CA 95403 (US); MCKINLEY, Laura, Santa Rosa, CA 95403 (US); TIEN, Tracey, Santa Rosa, CA 95403 (US)
(74) Representative: Zimmermann & Partner Patentanwälte mbB
(86) International application number: PCT/US2016/067426
(87) International publication number: WO 2017/112560

(56) References cited:
- US-A1- 2007 299 517
- US-A1- 2010 030 340
- US-A1- 2012 123 557
- US-A1- 2013 325 111

## Description

### FIELD

The present disclosed is directed toward methods of increasing the quality of biological tissue, such as biological tissue used with prosthetic heart valves. More particularly, it relates to methods of preparing dry cross-linked tissue with elevated compressibility.

### BACKGROUND

Biological prostheses or "bioprostheses" are devices derived at least partially from processed biological tissues to be used for implantation into humans. Examples of bioprostheses that are currently used or in development include heart valves, vascular grafts, ligament substitutes, pericardial patches and others.

Even though much is now known about biological tissue, bioprostheses and the processing, assembly, and performance thereof, there are still deficiencies that need to be overcome to provide a bioprostheses that preserves the native tissue properties while optimizing tissue biomechanics, minimizing calcification, and/or rendering the treated tissue hemocompatible.

For example, it is typically the case that after harvesting, the biological tissue must be stored under proper conditions, and in proper solutions, to preserve the native properties of the tissue prior to and during the tissue processing steps that are to be subsequently undertaken. In addition, the harvested biological tissue should be stored in a manner that mitigates or even reduces the bioburden of the harvested tissue.

Further, the primary component of biological tissues used to fabricate many bioprostheses is collagen, a term used herein a generic sense to refer to a family of related extracellular proteins. Collagen molecules assemble to form microfibrils, which in turn assemble into fibrils, resulting in collagen fibers.

The amino acids that make up the collagen molecules contain side groups that represent sites for potential chemical reaction on these molecules. Because collagenous tissues degrade rapidly upon implantation into a host recipient, it is necessary to stabilize the tissue if it is to be used for long-term clinical applications. Chemical stabilization by cross-linking collagen molecules within the tissue (also known as tissue fixation) is well-known, and glutaraldehyde is commonly used to cross-link tissue. US 2010/0030340 A1 describes plasticized grafts and methods of making and using same.

### SUMMARY

The scope of this invention is defined by the claims. Any "embodiment" or "example" which is disclosed in the description but is not covered by the claims should be considered as presented for illustrative purpose only. Some aspects of the present disclosure are directed to methods of preparing dry cross-linked biological tissue. A processing solution is prepared that contains a cross-linking agent and an organic water-soluble liquid. Biological tissue is immersed in the processing solution for an effective period of time to substantially displace water from the biological tissue and substantially cross-link the biological tissue to form dry cross-linked biological tissue. The effective period of time is between about 3 hours and about 24 hours.

Other aspects of the present disclosure are directed to methods of enhancing the compressibility of biological tissue used in a transcatheter heart valve. Biological tissue is decellularized. A processing solution is prepared that includes a cross-linking agent and an organic water-soluble liquid. The decellularized biological tissue is immersed in the processing solution for an effective period of time to substantially displace water from the biological tissue and substantially cross-link the biological tissue to form decellularized dry cross-linked biological tissue. The effective period of time is between about 3 hours and about 24 hours.

### BRIEF DESCRIPTION OF THE DRAWINGS

The accompanying drawings are included to provide a further understanding of embodiments and are incorporated in and constitute a part of this specification. The drawings illustrate embodiments and together with the description serve to explain principles of embodiments. Other embodiments and many of the intended advantages of embodiments will be readily appreciated as they become better understood by reference to the following detailed description. The elements of the drawings are not necessarily to scale relative to each other. Like reference numerals designate corresponding similar parts.
Fig. 1 is a photograph of a prosthetic heart valve including biological tissue prepared in accordance with principles of the present disclosure and following rehydration.
Fig. 2 is a photograph of a prosthetic heart valve including biological tissue prepared in accordance with principles of the present disclosure and following rehydration.
Fig. 3 is a photograph of a prosthetic heart valve including biological tissue prepared in accordance with conventional methods.
Fig. 4 is a graph comparing compressibility of biological tissue examples and comparative examples described in the Examples below.

### DETAILED DESCRIPTION

Some aspects of the present disclosure are directed to methods of preparing biological tissue for implantation that enhances the quality of the biological tissue, such as biological tissue used with a prosthetic heart valve. The present disclosure enhances the ability to handle bioprosthetic tissue because the dry and cross-linked biological tissue may be stored in dry conditions, which is in contrast to the aqueous solution that is typically used for storing biological tissue prior to implantation. The present disclosure can result in prepared biological tissue that grossly feels and qualitatively behaves like fully hydrated fixed tissue while being supple and having high tensile strength.

Examples of types of biological tissue that may be used with the present disclosure include porcine, bovine, equine, ovine, or other aortic or pulmonary valves and vascular tissues; human donor allografts; other sources of connective tissue matrices, including porcine, equine, ovine and other xenogeneic or allogeneic pericardial tissues; dura mater; omentum or other tissues of the digestive tract; skin, SIS (Small Intestinal Submucosa), placenta, uterus, or tissues reconstructed in vitro from cells from such tissues; and ocular tissues including cornea and sclera.

Examples of other bioprostheses or devices that may be formed from biological tissue processed as described herein include heart valves and valve leaflets; vascular grafts for peripheral, coronary and dialysis access; patches, strips, or buckles used to reinforce or repair soft tissues, hard tissues, cartilage, tendon, cornea, or the like for organ repair and reinforcement for effective reconstruction procedures (including native valve reconstruction, valve annuloplasty and repair).

The methods may also be applied to create structures or devices for tissue augmentation procedures (including cardiac wraps, bands, or reinforcements for congestive heart failure, vascular aneurysm repair and reinforcement including cerebral, aortic, and abdominal devices), and as an adjunct or support for other devices fabricated from synthetic materials such as DACRON® or PTFE; and scaffolds for repairing and/or regenerating tissues, either before or after implantation.

The drying and cross-linking of the biological tissue is achieved by immersing the biological tissue in a processing solution for an effective period of time, wherein the effective period of time is between about 3 hours and about 24 hours. After the biological tissue is immersed in the processing solution for the effective period of time, the biological tissue is substantially dry and substantially cross-linked.

According to the claimed invention, the processing solution contains a mixture of a cross-linking agent and one or more organic water-soluble liquids. Increasing the concentration of the organic water-soluble liquid in the processing solution decreases the amount of time that is needed to attain a particular reduction in the water concentration in the biological tissue.

In certain embodiments, the cross-linking agent may be an aldehyde that is capable of cross-linking the amine groups of the protein in the biological tissue. Examples of suitable aldehydes include glutaraldehyde and formaldehyde.

The cross-linking agent concentration in the processing solution is less than about 10 percent by volume. In certain embodiments, the cross-linking agent concentration in the processing solution is between about 0.10 percent by volume and about 1.00 percent by volume.

The organic water-soluble liquid can be a single organic water-soluble liquid or a combination of two or more organic water-soluble liquids. In some embodiments, the organic water-soluble liquid can include a polyhydric alcohol. Exemplary organic water-soluble liquids useful with the processing solutions of the present disclosure include, but are not limited to, glycerol, sucrose, propylene glycol, triethylene glycol, and tetraethylene glycol. The organic water-soluble liquid concentration in the processing solution is greater than about 5 percent by volume. In certain embodiments, the organic water-soluble liquid concentration in the processing solution is between about 10 percent by volume and about 60 percent by volume.

The processing solution can have a pH that is advantageous for biological tissue such as between about 7.3 and 7.5. The processing solution may also include a buffer that enhances the ability of the processing solution to remain at a pH that is advantageous for the biological tissue such as between about 7.3 and 7.5.

In certain embodiments, the buffer may be isotonic. The buffer may include at least one of sodium chloride, potassium phosphate monobasic, sodium phosphate dibasic heptahydrate, hydrochloric acid and sodium hydroxide. In other embodiments, the buffer includes a mixture of a phosphate buffered solution and a chelating agent.

The effective period of time in which the biological tissue is immersed in the processing solution may be affected by a variety of factors such as the concentration of the cross-linking agent and the organic water-soluble liquid in the processing solution and the temperature of the processing solution while the biological tissue is immersed therein.

The biological tissue is contacted with the processing solution for a time and at a temperature sufficient to permit the processing solution to penetrate into the interstices of the biological tissue and achieve substantial equilibrium between the processing solution and the fluids in the interstices of the biological tissue.

The time needed to achieve equilibrium is directly related to factors such as the thickness of the biological tissue and the concentration of the components in the processing solution. Additionally, the time needed to achieve equilibrium is inversely related to the ratio between the combined volume of the processing solution and the biological tissue to the rate at which the processing solution is mixed.

In particular, the effective period of time is inversely rated to the concentration of the cross-linking agent and the organic water-soluble liquid in the processing solution. As such, as the concentration of these components in the processing solution increases, the effective period of time decreases. According to the claimed invention, the effective period of time is between about 3 hours and about 24 hours.

During the effective time in which the biological tissue is immersed in the processing solution, the processing solution may be maintained at a temperature of no more than 60°C. In other embodiments, the processing solution may be maintained at a temperature of between about 15°C and about 40°C while the biological tissue is immersed in the processing solution. In still other embodiments, the processing solution may be maintained at a temperature of between about 17°C and about 37°C while the biological tissue is immersed in the processing solution.

At least one of the biological tissue and the processing solution may be agitated while the biological tissue is immersed in the processing solution. In certain embodiments, when the biological tissue and the processing solution are placed in an enclosed container, both of the biological tissue and the processing solution are simultaneously agitated.

Agitating at least one of the biological tissue and the processing solution enhances the amount of cross-linking agent and the organic water-soluble liquid that comes into contact with the biological tissue while the biological tissue is immersed therein.

The methods of the present disclosure may be conducted by placing the processing solution in an enclosed container. Using the enclosed container minimizes the evaporation of the components in the processing solution during the drying and cross-linking process.

A portion of the enclosed container that is not occupied by the processing solution and the immersed biological tissue may be filled with an inert gas. The inert gas may be selected from the group consisting of nitrogen, helium, neon, argon and combinations thereof.

In addition to drying and cross-linking the biological tissue, certain embodiments of the present disclosure also decellularize the biological tissue. Decellularization refers to the removal and/or extraction of cells and/or cellular components from a tissue. Using methods of the present disclosure, cellular components are removed from tissue by decellularization without damaging the structure and/or function of the tissue. Non-cellular components are retained in the tissue following decellularization using some methods of the present disclosure.

The phrase "cellular component," as used herein, refers to substances that constitute a portion of a cell, including cell membranes and macromolecules (e.g., nucleic acids, polypeptides, glycoproteins, etc.) that are normally found enclosed within a cell membrane, embedded within a cell membrane or attached to a cell membrane. The term does not include molecules that have been secreted by cells, e.g., extracellular matrix components such as collagen, elastin, proteoglycans, etc., even if such molecules are associated with or linked to the cell surface.

The term "nucleic acid" refers to deoxyribonucleotides or ribonucleotides and polymers thereof in either single- or double-stranded form, composed of monomers (nucleotides) containing a sugar, phosphate and a base which is either a purine or pyrimidine. Deoxyribonucleic acid (DNA) in the majority of organisms is the genetic material while ribonucleic acid (RNA) is involved in the transfer of information contained within DNA into proteins. The terms "nucleic acid," "nucleic acid molecule," "nucleic acid fragment," "nucleic acid sequence or segment," or "polynucleotide" may also be used interchangeably with gene, cDNA, DNA and RNA encoded by a gene.

The terms "polypeptide," "protein," and "peptide" are used interchangeably herein. It is well-known in the art of protein biochemistry that amino acids, the 'building blocks' of proteins, have particular sizes and characteristics, such as charge, hydrophobicity and hydrophilicity. For example, nonpolar (hydrophobic) amino acids include alanine, leucine, isoleucine, valine, proline, phenylalanine, tryptophan, and tyrosine. Polar neutral amino acids include glycine, serine, threonine, cysteine, tyrosine, asparagine and glutamine. The positively charged (basic) amino acids include arginine, lysine and histidine. The negatively charged (acidic) amino acids include aspartic acid and glutamic acid.

By "fragment" or "portion" is meant a full length or less than full length of the nucleotide sequence encoding, or the amino acid sequence of, a polypeptide or protein.

By "non-cellular component" is meant a substance present within a biological tissue (either a native tissue or a tissue-engineered construct) that was derived from a cell or was present within the tissue but is not contained within the plasma membrane of a cell. Examples of such components include extracellular matrix (ECM) components (e.g., elastin, collagen (Type I, IV), laminin, fibronectin, etc.) and the like.

"Native" refers to naturally occurring. Thus, by "native tissue protein," for example, is meant a protein that is present in naturally occurring tissue, i.e., tissue from a mammal that remains substantially intact and substantially retains the structure naturally found within the body of the mammal.

The terms "antigen" and "immunogen" are used interchangeably herein, and refer to any substance that elicits an immune response when administered to a mammal.

Decellularization may be achieved using a variety of detergents, emulsification agents, proteases, and/or high or low ionic strength solutions. In certain embodiments of the present disclosure, decellularization is performed under conditions and for sufficient times such that antigens, e.g., immunogenic cellular components including lipid membranes, membrane associated antigens and soluble proteins present in the tissue sample, are substantially removed, while the structural and mechanical integrity of ECM proteins, e.g. collagen and elastin, are maintained.

In particular, the decellularization solution facilitates the breakdown of the cellular structure in the tissue sample for the removal of cells, as well as cell debris and cell organelles from the ECM, and proteins, by breaking up the phospholipid bilayer of cell membranes. The decellularization solution contains at least one decellularization agent that is capable of associating, e.g., attaching permanently or temporarily via a covalent bond, ionic bond, hydrogen bond or van der Waals forces, so as to form a complex with macromolecules, e.g., proteins, including immunogenic or antigenic proteins, and/or nucleic acids, that are present on or within the cell and/or cell membranes.

The decellularization solutions of the present disclosure may not cause gross alteration in the structure of the tissue sample, the ECM of the tissue sample, or in its biomechanical properties. The effects of decellularization on structure may be evaluated by known techniques, including light microscopy, ultrastructural examination, histology, electron microscopy, calorimetry, etc. In addition, biomechanical tests, which are well known in the art, may be used to evaluate the effects of various decellularization protocols on tissue properties. Selection and interpretation of such tests will depend, in general, upon the nature of the construct and the purpose for which it is intended.

The concentration of the decellularization agent used to decellularize the tissue varies depending on the desired requirements. In certain embodiments, the total concentration of decellularization agent added to the solution can be about 0.01% to about 10% by weight. The total concentration of all decellularization agents added to the decellularization solution can be about 2 weight percent or less in some embodiments. For example, the total concentration of decellularization agent(s) in some methods is between about 0.25 percent and about 2 percent (weight by volume or volume by volume) between about 0.5 percent and about 1 percent.

In certain embodiments, the decellularization solution can also contain between about 0.1% and 1% saline by weight and between about 0.025% and 0. 1% by weight sodium azide.

The tissue can be placed in the decellularization solution for periods of up to about 7 days. In other embodiments, the tissue is placed in the decellularization solution for about 70 hours, about 60 hours, about 50 hours or about 40 hours or less, depending on the embodiment and depending on the tissue thickness, and/or tissue density.

The tissue-contacting period can be long enough to allow non-structural proteins and/or antigens associating, e.g., forming protein-detergent complexes, present in the tissue with the detergent. In one method, the tissue is placed in contact with the decellularization solution for at least about 40 hours. In another method, tissue about 2 mm thick is placed in contact with the decellularization solution for about 50 hours.

Tissue constructs from tissue culture may require only about 2 days or less depending on the construct. Denser tissue may require longer contact times. For example, femoral artery tissue may require a longer contact time than femoral vein tissue.

The tissue can be subjected to mechanical processing during decellularization by any number of methods. For example, a roller bottle apparatus or an apparatus that applies a rolling-type agitation can be employed to keep the treated tissue suspended during treatment.

The tissue and decellularization solution can be maintained at a temperature of between about 20 to 40°C during the decellularization process.

Decellularized tissue can then be rinsed using standard protocols or by methods described herein to substantially remove the decellularization agent from the tissue thickness center.

Once the decellularization is complete, the biological tissue is subjected to the drying and cross-linking steps that are discussed in more detail above (i.e. simultaneously drying and fixing (and optionally sterilizing) the tissue in one single step). This process thereby produces biological tissue that is ready for use or which is suitable for storing in dry conditions prior to use (e.g., the biological tissue resulting from some methods of the present disclosure can be assembled into an implantable device, such as a prosthetic heart valve, and the implantable device can be stored in a dry condition prior to implant).

The biological tissue exhibits enhanced performance characteristics when compared to prior implantable biological tissue such as enhanced compressibility and enhanced recoverability. These characteristics enhance the ability to use the biological tissue in a variety of applications.

Compressibility of dry decellularized (or "decell") tissue resulting from some methods of the present disclosure is at least about 30 percent greater than the compressibility of the same biological tissue processed by other methods that result in dry non-decell tissue. In certain embodiments, the compressibility of the dry decell tissue is about 50 greater than the compressibility of dry non-decell tissue.

Additionally, the dry decell tissue resulting from some methods of the present disclosure has enhanced compressibility when compared to biological tissue processed in accordance with conventional methods to serve as bioprosthetic tissue of an implantable device and desired to be stored in an aqueous solution (i.e., non-dry), such as the bioprosthetic tissue used with many currently-available prosthetic heart valves. As a point of reference, one conventional method for preparing bioprosthetic tissue for use with a prosthetic heart valve includes received pericardial tissue from a slaughterhouse, cleaning the tissue, then fixing the tissue in a solution of approximately 0.25% glutaraldehyde for a minimum of 24 hours at room temperature (referenced throughout this disclosure as "conventional, non-decell method" or "conventionally prepared non-decell biological tissue"). In certain embodiments, the compressibility of the dry decell tissue resulting from some methods of the present disclosure is at least about 5 percent greater than the compressibility of conventionally prepared non-decell bioprosthetic tissue utilized with conventional prosthetic heart valves and stored in an aqueous solution. In other embodiments, the compressibility of the dry decell tissue is at least about 10 percent greater than the compressibility of conventionally prepared non-decell biological tissue utilized with prosthetic heart valves stored in an aqueous solution.

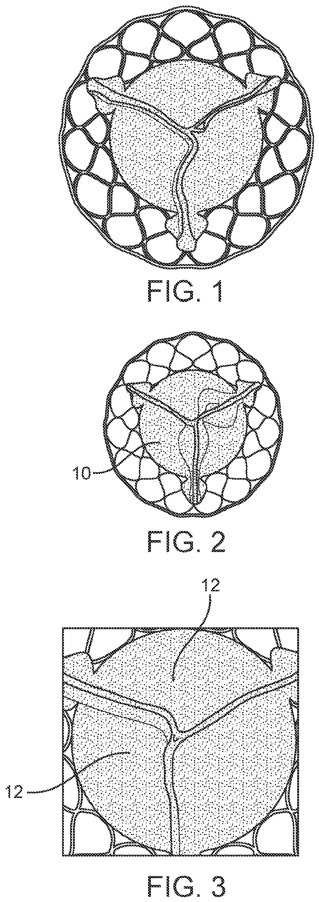

Another aspect related to the properties of the biological tissue resulting from some methods of the present disclosure is improved imprint recoverability after the biological tissue has been rehydrated (e.g., following implantation, the prepared biological tissue of a prosthesis, such as the prepared biological tissue of a prosthetic heart valve, rehydrates in the presence of bodily fluids). Set forth in Fig. 1 is an image of a prosthetic heart valve incorporating bioprosthetic tissue leaflets prepared in accordance with methods of the present disclosure (e.g., dry decell tissue), following deployment and rehydration. The photograph of Fig. 1 reflects that the dry decell tissue (post deployment of the prosthetic heart valve and rehydration) exhibits no imprints. Set forth in Fig. 2 is an image of a prosthetic heart valve incorporating bioprosthetic tissue leaflets prepared using drying techniques of the present disclosure but without the optional decellularization methods of the present disclosure, following deployment and rehydration. The photograph of Fig. 2 reflects that the dry, non-decell tissue (post deployment of the prosthetic heat valve and rehydration) exhibits some imprints (identified at 10 in Fig. 2). Set forth in Fig. 3 is an image of a prosthetic heart valve incorporating conventionally prepared non-decell bioprosthetic tissue leaflets (prepared using a conventional method in which pericardial tissue that is received from a slaughterhouse is cleaned and then fixed in a low concentration (approximately 0.25%) of glutaraldehyde for a minimum of 24 hours at room temperature), including storage prior to use in an aqueous solution, following deployment (it being understood that because the prosthetic heart valve of Fig. 3 is hydrated prior to use, "rehydration" is not applicable). The photograph of Fig. 3 reflects that the conventionally prepared bioprosthetic tissue exhibits imprints (identified at 12 in Fig. 3) post deployment. A comparison of Figs. 1-3 reveals that the rehydrated decell tissue prepared in accordance with methods of the present disclosure exhibits no imprints post deployment or rehydration.

The product and method of the present disclosure are described in the following examples. These examples are provided as an illustration and are not intended to limit the present disclosure.

### EXAMPLES

The compressibility of tissue prepared according to some methods of the present disclosure was evaluated in comparison to the compressibility of conventionally prepared non-decell biological tissue. In particular, an example tissue sample was prepared by fresh porcine pericardial tissue in a detergent solution at room temperature for 96 hours to decellularize the tissue; the decellularized tissue was then placed in a buffered solution of 0.2% glutaraldehyde and 25% glycerol at room temperature for 24 hours to simultaneously dry and fix the example tissue sample. The example tissue sample was then air dried. Immediately prior to the compression testing described below, the example tissue sample was immersed in a normal saline solution for 1 hour to rehydrate the example tissue sample. A comparative example tissue sample was provided as conventionally prepared, non-decell biotissue, prepared from fresh porcine pericardial tissue that was cleaned, fixed in a solution of 0.25% glutaraldehyde for at least 24 hours at room temperature, and then stored in a glutaraldehyde solution.

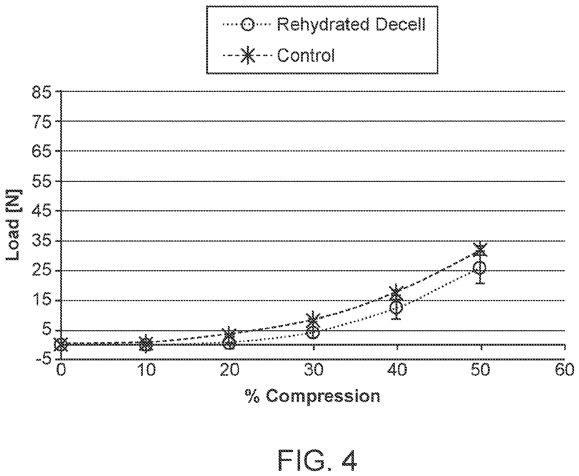

To evaluate compressibility, a load was placed on similarly sized example and comparative example tissue samples and the compression was recorded at different loads. The results of this evaluation are reported in Fig. 4. In the plot of Fig. 4, compression results for the example tissue sample is recorded as "Rehydrated Decell", and the compression results for the comparative tissue sample is recorded as "Control".

The compression of the rehydrated decell sample was superior to the comparative example (or "Control") sample. In particular, the load to achieve 50% compression of the rehydrated decell sample was decreased by more than 10% as compared to the compression of the Control sample.

In the preceding detailed description, reference is made to the accompanying drawings, which form a part hereof, and in which is shown by way of illustration specific embodiments in which the features of the present disclosure may be practiced. In this regard, directional terminology, such as "top," "bottom," "front," "back," "leading," "trailing," etc., is used with reference to the orientation of the Figure(s) being described. Because components of embodiments can be positioned in a number of different orientations, the directional terminology is used for purposes of illustration and is in no way limiting. It is to be understood that other embodiments may be utilized and structural or logical changes may be made without departing from the scope of the present disclosure. The preceding detailed description, therefore, is not to be taken in a limiting sense, and the scope of the present invention is defined by the appended claims.

## Claims

1. A method of preparing dry cross-linked biological tissue comprising:
preparing a processing solution that comprises a cross-linking agent and an organic water-soluble liquid; and
immersing a biological tissue in the processing solution for an effective period of time to substantially displace water from the biological tissue and substantially cross-link the biological tissue to form dry cross-linked biological tissue,
wherein the effective period of time is between about 3 hours and about 24 hours.

2. The method of claim 1, wherein the cross-linking agent is provided in the processing solution at a concentration of between about 0.10 percent by weight and about 1.00 percent by weight, or wherein the cross-linking agent comprises at least one of an amine- and a carboxyl-reactive crosslinker.

3. The method of claim 1, wherein the cross-linking agent includes glutaraldehyde.

4. The method of claim 1, wherein the organic water-soluble liquid is provided in the processing solution at a concentration of between about 10 percent by volume and about 70 percent by volume.

5. The method of claim 1, wherein the organic water-soluble liquid comprises at least one polyhydric alcohol, or wherein the organic water-soluble liquid includes at least one of glycerol, sucrose, propylene glycol, triethylene glycol, and tetraethylene glycol.

6. The method of claim 1, wherein the processing solution is saline buffered.

7. The method of claim 1, wherein the biological tissue is fresh prior to being immersed in the processing solution.

8. The method of claim 1, and further comprising agitating at least one of the biological tissue and the processing solution while the biological tissue is immersed in the processing solution, or further comprising maintaining the processing solution at a temperature of between about 15°C and about 40°C while the biological tissue is immersed in the processing solution.

9. The method of claim 1, and further comprising applying a force to the biological tissue while the biological tissue is immersed in the processing solution.

10. The method of claim 1, and further comprising decellularizing the biological tissue before the step of immersing in the processing solution, and further wherein following the step of immersing, the biological tissue is decellularized dry cross-linked biological tissue.

11. The method of claim 10, wherein decellularization removes a substantial amount of the cells or cellular components from the biological tissue, or, wherein decellularization comprises immersing the biological tissue in at least one detergent, emulsification agent, protease, high ionic strength solution, low ionic strength solution or combination thereof.

12. The method of claim 1, further comprising air drying the dry cross-linked biological tissue.

13. The method of claim 1, wherein the dry cross-linked biological tissue is acceptable for use in at least one of a prosthetic heart valve, a prosthetic valve leaflet, a vascular graft, and an implantable treatment device to treat soft tissue, hard tissue, cartilage, tendon, cornea, or an organ.

14. The method of claim 1, and further comprising storing the dry cross-linked biological tissue in non-liquid environment.

15. The method of claim 10 , and further comprising:
compressing the decellularized dry cross-linked biological tissue; and
assembling the compressed decellularized dry cross-linked biological tissue to a stent frame of a transcatheter heart valve.

## Patentansprüche

1. Verfahren zum Herstellen von trockenem vernetztem biologischem Gewebe, umfassend:
Herstellen einer Verarbeitungslösung, die ein Vernetzungsmittel und eine organische wasserlösliche Flüssigkeit umfasst; und
Eintauchen eines biologischen Gewebes in die Verarbeitungslösung für einen wirksamen Zeitraum, um Wasser aus dem biologischen Gewebe im Wesentlichen zu verdrängen und das biologische Gewebe im Wesentlichen zu vernetzen, um trockenes vernetztes Gewebe zu bilden,
wobei der wirksame Zeitraum zwischen etwa 3 Stunden und etwa 24 Stunden beträgt.

2. Verfahren nach Anspruch 1, wobei das Vernetzungsmittel in der Verarbeitungslösung in einer Konzentration zwischen etwa 0,10 Gew.-% und etwa 1,00 Gew.-% bereitgestellt wird, oder wobei das Vernetzungsmittel einen amin- und/oder einen carboxylreaktiven Vernetzer umfasst.

3. Verfahren nach Anspruch 1, wobei das Vernetzungsmittel Glutaraldehyd beinhaltet.

4. Verfahren nach Anspruch 1, wobei die organische wasserlösliche Flüssigkeit in der Verarbeitungslösung in einer Konzentration zwischen etwa 10 Volumenprozent und etwa 70 Volumenprozent bereitgestellt wird.

5. Verfahren nach Anspruch 1, wobei die organische wasserlösliche Flüssigkeit mindestens einen mehrwertigen Alkohol umfasst, oder wobei die organische wasserlösliche Flüssigkeit Glyzerin, Saccharose, Propylenglykol, Triethylenglykol und/oder Tetraethylenglykol beinhaltet.

6. Verfahren nach Anspruch 1, wobei die Verarbeitungslösung mit Kochsalzlösung gepuffert ist.

7. Verfahren nach Anspruch 1, wobei das biologische Gewebe vor dem Eintauchen in die Verarbeitungslösung frisch ist.

8. Verfahren nach Anspruch 1, ferner umfassend ein Bewegen des biologischen Gewebes und/oder der Verarbeitungslösung, während das biologische Gewebe in die Verarbeitungslösung eingetaucht ist, oder ferner umfassend ein Halten der Verarbeitungslösung bei einer Temperatur zwischen etwa 15 °C und etwa 40 °C, während das biologische Gewebe in die Verarbeitungslösung eingetaucht ist.

9. Verfahren nach Anspruch 1, und ferner umfassend ein Ausüben einer Kraft auf das biologische Gewebe, während das biologische Gewebe in die Verarbeitungslösung eingetaucht ist.

10. Verfahren nach Anspruch 1, und ferner umfassend ein Dezellularisieren des biologischen Gewebes vor dem Schritt des Eintauchens in die Verarbeitungslösung, und ferner wobei nach dem Schritt des Eintauchens das biologische Gewebe dezellularisiertes trockenes vernetztes biologisches Gewebe ist.

11. Verfahren nach Anspruch 10, wobei die Dezellularisierung eine wesentliche Menge der Zellen oder zellulären Komponenten aus dem biologischen Gewebe entfernt, oder wobei die Dezellularisierung das Eintauchen des biologischen Gewebes in mindestens ein Detergens, ein Emulgiermittel, eine Protease, eine Lösung mit hoher lonenstärke, eine Lösung mit niedriger lonenstärke oder eine Kombination davon umfasst.

12. Verfahren nach Anspruch 1, ferner umfassend ein Lufttrocknen des trockenen vernetzten biologischen Gewebes.

13. Verfahren nach Anspruch 1, wobei das trockene vernetzte biologische Gewebe zur Verwendung in einer prothetischen Herzklappe, einem prothetischen Klappensegel, einem Gefäßtransplantat und/oder einer implantierbaren Behandlungsvorrichtung zum Behandeln von Weichgewebe, Hartgewebe, einem Knorpel, einer Sehne, Hornhaut oder einem Organ geeignet ist.

14. Verfahren nach Anspruch 1, und ferner umfassend ein Lagern des trockenen vernetzten Gewebes in nicht-flüssiger Umgebung.

15. Verfahren nach Anspruch 10, und ferner umfassend:
Komprimieren des dezellularisierten trockenen vernetzten Gewebes; und
Anordnen des komprimierten dezellularisierten trockenen vernetzten Gewebes an einem
Stentrahmen einer Transkatheterherzklappe.

## Revendications

1. Procédé de préparation d'un tissu biologique réticulé sec, comprenant :
la préparation d'une solution de traitement qui comprend un agent de réticulation et un liquide organique soluble dans l'eau ; et
l'immersion d'un tissu biologique dans la solution de traitement pendant une période de temps efficace pour repousser sensiblement l'eau du tissu biologique et réticuler sensiblement le tissu biologique de façon à former un tissu biologique réticulé sec,
la période de temps efficace étant comprise entre environ 3 heures et environ 24 heures.

2. Procédé selon la revendication 1, dans lequel l'agent de réticulation est fourni dans la solution de traitement à une concentration comprise entre environ 0,10 pour cent en poids et environ 1,00 pour cent en poids, ou dans lequel l'agent de réticulation comprend un agent de réticulation réactif à une amine et un agent de réticulation réactif au carboxyle.

3. Procédé selon la revendication 1, dans lequel l'agent de réticulation comprend du glutaraldéhyde.

4. Procédé selon la revendication 1, dans lequel le liquide organique soluble dans l'eau est fourni dans la solution de traitement à une concentration comprise entre environ 10 pour cent en volume et environ 70 pour cent en volume.

5. Procédé selon la revendication 1, dans lequel le liquide organique soluble dans l'eau comprend au moins un alcool polyhydrique, ou dans lequel le liquide organique soluble dans l'eau comprend du glycérol et/ou du saccharose et/ou du propylène glycol et/ou du triéthylène glycol et/ou du tétraéthylène glycol.

6. Procédé selon la revendication 1, dans lequel la solution de traitement est tamponnée avec une solution saline.

7. Procédé selon la revendication 1, dans lequel le tissu biologique est frais avant son immersion dans la solution de traitement.

8. Procédé selon la revendication 1, comprenant en outre l'agitation du tissu biologique et/ou de la solution de traitement pendant l'immersion du tissu biologique dans la solution de traitement, ou comprenant en outre le maintien de la solution de traitement à une température comprise entre environ 15 °C et environ 40 °C pendant l'immersion du tissu biologique dans la solution de traitement.

9. Procédé selon la revendication 1, comprenant en outre l'application d'une force sur le tissu biologique pendant l'immersion du tissu biologique dans la solution de traitement.

10. Procédé selon la revendication 1, comprenant en outre la décellularisation du tissu biologique avant l'étape d'immersion dans la solution de traitement, et en outre dans lequel, après l'étape d'immersion, le tissu biologique est un tissu biologique réticulé sec décellularisé.

11. Procédé selon la revendication 10, dans lequel la décellularisation élimine une quantité substantielle de cellules ou de composants cellulaires du tissu biologique, ou dans lequel la décellularisation comprend l'immersion du tissu biologique dans un détergent et/ou un agent émulsifiant et/ou une protéase et/ou une solution à force ionique élevée et/ou une solution à force ionique faible ou une combinaison de ceux-ci.

12. Procédé selon la revendication 1, comprenant en outre le séchage à l'air du tissu biologique réticulé sec.

13. Procédé selon la revendication 1, dans lequel le tissu biologique réticulé sec est acceptable dans une valvule cardiaque prothétique et/ou un feuillet de valvule prothétique et/ou une greffe vasculaire et/ou un dispositif de traitement implantable permettant de traiter d'un tissu mou, d'un tissu dur, d'un cartilage, d'un tendon, d'une cornée ou d'un organe.

14. Procédé selon la revendication 1, comprenant en outre la conservation du tissu biologique réticulé sec dans un environnement non liquide.

15. Procédé selon la revendication 10, comprenant en outre :
la compression du tissu biologique réticulé sec décellularisé ; et
l'assemblage du tissu biologique réticulé sec décellularisé comprimé pour former un cadre d'endoprothèse d'une valvule cardiaque transcathéter.
